# EUROPÄISCHE PATENTSCHRIFT

(11) **EP 3 069 760 B1**
(45) Veröffentlichungstag und Bekanntmachung des Hinweises auf die Patenterteilung: **29.04.2020**
(21) Anmeldenummer: 16158393.5
(22) Anmeldetag: 03.03.2016
(51) Int. Cl.: A61N 1/375, A61N 1/36

(54) **IMPLANTIERBARE ELEKTROMEDIZINISCHE VORRICHTUNG**
IMPLANTABLE ELECTROMEDICAL DEVICE
DISPOSITIF ELECTROMEDICAL IMPLANTABLE

(30) Priorität: 20.03.2015 US 201562135712 P
(43) Veröffentlichungstag der Anmeldung: 21.09.2016
(73) Patentinhaber: BIOTRONIK SE & Co. KG, 12359 Berlin (DE)
(72) Erfinder: Pscherer, Norbert, 90766 Fürth (DE); Kalb, Hermann, 91052 Erlangen (DE); Haas, Erich, 91604 Flachslanden (DE); Seidler, Sahika, 90461 Nürnberg (DE)
(74) Vertreter: Galander, Marcus

(56) Entgegenhaltungen:
- US-A1- 2011 048 770
- US-A1- 2011 232 961
- US-A1- 2012 194 981

## Beschreibung

Die Erfindung betrifft eine implantierbare elektromedizinische Vorrichtung, umfassend ein Umhüllungsteil, das eine elektronische oder elektrische Funktionseinheit aufnimmt, eine an der Außenseite des Umhüllungsteils angeordnete Elektrode oder einen Leitungsanschluss und eine Durchführung, die eine Öffnung des Umhüllungsteils umschließt und mindestens ein die Elektrode oder den Leitungsanschluss mit der Funktionseinheit verbindendes metallisches Leiterelement aufnimmt und einen metallischen Umfangsflansch hat.

Derartige Geräte sind insbesondere als Herzschrittmacher oder implantierbare Kardioverter (speziell Defibrillatoren) seit langem in massenhaftem Einsatz. Es kann sich hierbei aber auch um eine weniger komplexe Vorrichtung, wie etwa eine Elektroden- oder Sensorleitung oder auch ein Cochlearimplantat, handeln.

Die meisten praktisch bedeutsamen implantierbaren elektromedizinischen Geräte sind dazu vorgesehen, über geeignet platzierte Elektroden elektrische Impulse an reizbares Körpergewebe abzugeben. Um diese Funktion auszuführen, sind im Gehäuse des Gerätes elektronische/elektrische Funktionseinheiten zur Erzeugung der Impulse und zur geeigneten Steuerung der Impulserzeugung untergebracht, und außen am Gerät sind unmittelbar Elektroden oder Anschlüsse für mindestens eine Elektrodenleitung vorgesehen, in deren distalem Endabschnitt die Elektroden zur Impulsübertragung an das Gewebe angebracht sind. Die elektronischen/elektrischen Funktionseinheiten im Geräteinneren sind in einer Weise mit den äußeren Elektroden oder Elektrodenleitungsanschlüssen zu verbinden, die unter den speziellen Bedingungen des implantierten Zustandes eine absolut und dauerhaft verlässliche Funktion gewährleistet.

Bekannt sind insbesondere Durchführungen, deren Grund- und Isolationskörper im Wesentlichen aus Keramik oder Glas besteht, wobei auch mehrschichtige bzw. mehrteilige Aufbauten unter Einsatz von Metallen oder Metalloxiden entwickelt wurden und eingesetzt werden. Derartige bekannte Durchführungen erfüllen weitgehend die an sie gestellten Anforderungen.

Die bekannten Durchführungs-Aufbauten sind jedoch in der Herstellung relativ arbeits- und somit kostenaufwändig. Insbesondere sind mehrere Arbeits- und Prozessschritte erforderlich, es ist ein aufwändiges Fügen von mehreren Einzelkomponenten und teilweise eine Vorbehandlung derselben (z.B. Reinigung, Oberflächenvorbehandlung, Herstellung der Lötbarkeit) erforderlich, und die Prozesse müssen dementsprechend auch mehrere Prüfschritte umfassen.

Aus der DE 10 2009 003 958 B4 ist ein Verfahren zur Herstellung einer Durchführung eines Gerätes bekannt, bei dem ein metallischer Leiter mit einer isolierenden Ummantelung (speziell aus einem Oxid des Leiters) versehen und auf diesen isolierten Leiter ein Flansch mittels Metall-Pulverspritzgießen mit anschließender Abkühlung aufgeschrumpft wird. Aus der EP 1 820 534 A2 ist es auch bekannt, einen Umfangsflansch um eine ansonsten aus mehreren Teilen vormontierte Durchführung durch das Verfahren des Metall-Pulverspritzgießens zu erzeugen. Aus der US 2008/0209723 A1 ist es auch bekannt, ein Keramikbauteil in der Verarbeitungsstufe des sogenannten Bräunlings oder Grünlings auf einen metallischen Leiterstift aufzuschrumpfen.

Aus der US 2012/0319319 A1 ist es bekannt, Durchführungen für hermetisch abgedichtete Gerätegehäuse zu erzeugen, indem sowohl ein Isolationskörper als auch ein Leiter, der diesen durchläuft, mittels eines Pulverspritzgießverfahrens gebildet werden.

Die US 2011/0048770 A1 offenbart eine implantierbare medizinische Vorrichtung gemäß dem Oberbegriff des Anspruchs 1.

Der Erfindung liegt die Aufgabe zu Grunde, ein verbessertes implantierbares elektromedizinisches Gerät bereitzustellen, welches kostengünstig herstellbar und hochgradig zuverlässig ist.

Diese Aufgabe wird in ihrem Vorrichtungsaspekt durch ein Gerät mit den Merkmalen des Anspruchs 1 und in ihrem Verfahrensaspekt durch ein Verfahren mit den Merkmalen des Anspruchs 9 gelöst. Zweckmäßige Fortbildungen des Erfindungsgedankens sind Gegenstand der abhängigen Ansprüche.

Die Erfindung geht von dem Gedanken eines Einsatzes der MIM-Technologie für die metallischen Komponenten (Pin) und Flansch einer Durchführung zur Herstellung einer Durchführung in einem Prozessschritt aus. Der Prozess lässt zu, dass sowohl der außen liegende Flansch als auch der innen liegende Pin durch eine bestimmte Geometrie beim Schrumpfen eine feste mechanisch-chemische Verbindung mit dem Isolator eingehen. Das Schrumpfverhalten der eingesetzten Komponenten kann durch Vorsintern (z.B. Bräunling) oder durch eine bestimmte Zusammensetzung des Spritzpulvers (Anteil des Binders) für das gemeinsame Sintern aufeinander abgestimmt werden. Dies gilt auch für den physiko-chemischen Beitrag des Verfahrens zur Schaffung einer festen Verbindung der einzelnen Teile.

Das vorgeschlagene Verfahren bietet, zumindest in bevorzugten Ausführungen, einen oder mehrere der folgenden Vorteile:
- hohe Wiederholgenauigkeit + Positioniergenauigkeit
- kein gesonderter Fügeprozess für Einzelkomponenten erforderlich
- kein Lot erforderlich
- kostengünstig bei hohen Stückzahlen
- geringe Bestückungsaufwand
- starke Reduktion der Fertigungshilfsmittel
- weniger Prüf- und Vorbehandlungsschritte (Prozessschritte) notwendig.

In einer Ausführung der Erfindung sind der Umfangsflansch und das oder jedes Leiterelement als materialeinheitlich durch Metall-Pulverspritzgießen gebildete Sinterteile ausgeführt. Grundsätzlich ist es auch möglich, den Umfangsflansch und die Leiterelemente aus verschiedenen Materialien, aber jeweils durch das erwähnte Verfahren, zu bilden. Dies erfordert jedoch eine komplexere Prozessführung und ist daher aus derzeitiger Sicht weniger bevorzugt.

In einer vorteilhaften Ausgestaltung ist an dem oder jedem Leiterelement je ein derart direkt an einer vorrichtungs-inneren und einer vorrichtungs-äußeren Oberfläche oder einer dort angeordneten Ausnehmung des Keramik-Grundgehäuses angeordneter Fortsatz vorgesehen. Noch spezieller sind zusätzlich am Umfangsflansch an einer vorrichtungs-inneren und einer vorrichtungs-äußeren Oberfläche oder einer dort angeordneten Ausnehmung des Keramik-Grundgehäuses angeordnete Fortsätze vorgesehen.

Das Anpressen geschieht im Prozess durch das Abkühlen der heiß geformten Teile Umfangsflansch und Leiterelement, welches zu einem Zusammenziehen derselben in Dickenrichtung des Keramik-Grundkörpers bzw. zu einem Zusammenziehen des Umfangsflansches in der Richtung normal zur Grenzfläche Umfangsflansch-Keramikmantelfläche führt. In weiter vorteilhafter Ausgestaltung ist hierbei der oder jeder Fortsatz ring- oder scheibenförmig den Innenumfang des Umfangsflansches bzw. den Außenumfang des oder jedes Leiterelements umgebend ausgebildet. Hierdurch wird die jeweilige Grenzfläche mit dem Keramik-Grundkörper vollumfänglich abgedeckt und somit insbesondere ohne zusätzliche Hilfsmittel hermetisch dicht.

In einer weiteren Ausführung der Erfindung ist der Keramik-Grundkörper im Wesentlichen aus Aluminiumoxid-Keramik gebildet; grundsätzlich sind aber auch andere technische Keramiken einsetzbar. Des Weiteren sind bevorzugt der Umfangsflansch und/oder das oder jedes Leiterelement aus Titan oder einer Titanlegierung gebildet. Grundsätzlich können aber auch verwandte Metalle, wie Molybdän, Tantal, Wolfram, Vanadium, Zirkon oder Iridium und Legierungen derselben oder Nickel oder Palladium oder deren Legierungen zum Einsatz kommen.

Unter verfahrenstechnischen Gesichtspunkten umfasst eine zweckmäßige Ausführung der Erfindung die Schritte:
- Bilden des Keramik-Grundkörpers,
- Bereitstellen einer Pulverspritzgussform,
- Einlegen des Keramik-Grundkörpers in teilweise oder vollständig vorgesintertem Zustand in die Pulverspritzgussform,
- Einspritzen von Spritzgießmaterial zur Bildung des Umfangsflansches und des oder jedes Leiterelementes in die Pulverspritzgussform,
- Sintern/Wärmebehandlung der Pulverspritzgussform mit dem darin aufgenommenen Material zum Entbinden, Ausgasen und Schrumpfen des den Umfangsflansch und das oder jedes Leiterelement bildenden Materials und
- Entnehmen der Durchführung aus der Pulverspritzgussform.

Üblicherweise wird das Verfahren noch den späteren Schritt eines Verbindens der Durchführung mit der Umhüllung, insbesondere durch Verschweißen oder Verkleben oder Aufschrumpfen der Umhüllung auf den Umfangsflansch der Durchführung umfassen. Die konkrete Art der Montage der Durchführung hängt selbstverständlich vom Typ des Gerätes ab, in dem sie eingesetzt wird.

In einer ersten wichtigen Anwendung der Erfindung ist das Gerät ausgebildet als Elektrostimulationsgerät, insbesondere Herzschrittmacher oder Kardioverter, wobei das Umhüllungsteil als Gerätegehäuse ausgestaltet und die Elektrode oder der Leitungsanschluss an einem gegenüber dem Gerätegehäuse separaten Kopfteil angeordnet ist und die Durchführung zwischen dem Gerätegehäuse und dem Kopfteil angeordnet ist. In einer weiteren wichtigen Anwendung handelt es sich um eine Elektrodenleitung, wobei insbesondere das Leiterelement in seinem umhüllungs-äußeren Abschnitt zugleich eine Elektrode bildet.

Vorteile und Zweckmäßigkeiten der Erfindung ergeben sich im Übrigen aus der Beschreibung von Ausführungsbeispielen anhand der Figuren. Von diesen zeigen:
- Fig. 1: eine schematische, teilweise geschnittene Darstellung eines implantierbaren elekt-romedizinischen Geräts,
- Fig. 2: eine perspektivische Darstellung einer beispielhaften Durchführung des Gerätes nach Fig. 1,
- Fig. 3: eine Längsschnittdarstellung dieser beispielhaften Durchführung und
- Fig. 4: eine Längsschnittdarstellung eines weiteren Ausführungsbeispiels der Erfindung.

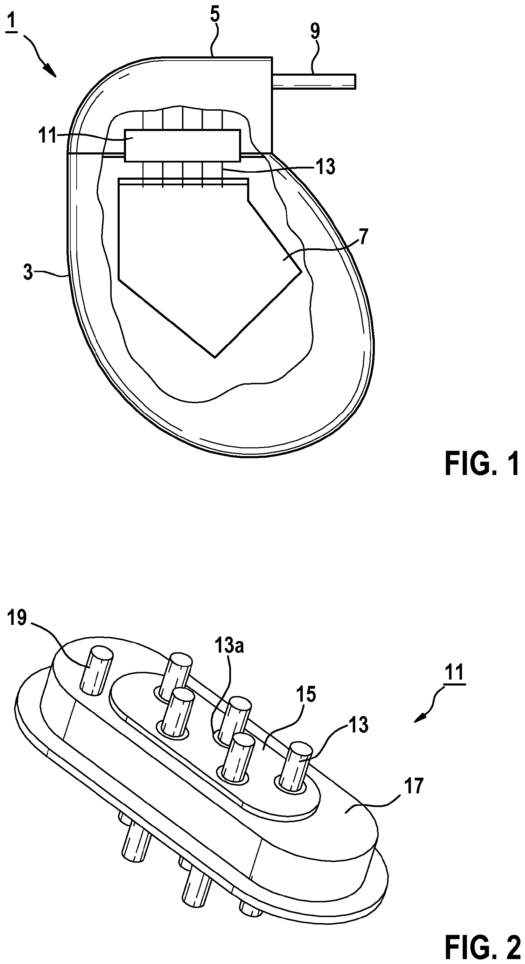

Fig. 1 zeigt einen Herzschrittmacher 1 mit einem Schrittmachergehäuse 3 und einem Kopfteil (Header) 5, in dessen Innerem neben anderen elektronischen Komponenten eine Leiterplatte (PCB) 7 angeordnet und mit dessen im Header angeordneten (nicht gezeigten) Leitungsanschluss eine Elektrodenleitung 9 verbunden ist. Eine zwischen Gerätegehäuse 3 und Header 5 vorgesehene Durchführung 11 umfasst eine Mehrzahl von Anschlussstiften 13. Die Anschlussstifte sind an einem Ende durch eine entsprechende Bohrung in der Leiterplatte gesteckt und mit dieser weich verlötet.

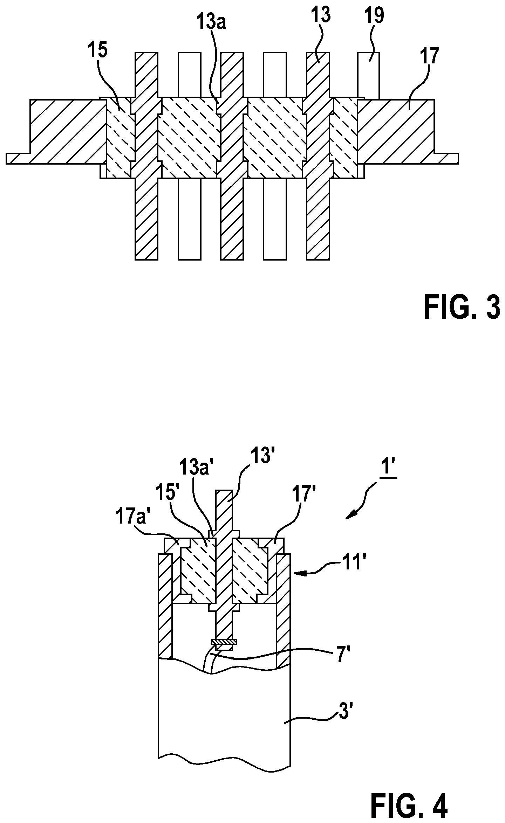

Fig. 2 zeigt in einer perspektivischen Darstellung ein Ausführungsbeispiel der Durchführung 11, deren Bestandteile neben den Anschlussstiften 13 ein Keramik-Grundkörper (Isolator) 15 und ein metallischer Umfangsflansch 17 sind. In den Umfangsflansch 17 ist ein Massepin 19 eingefügt. Fig. 3 zeigt deren Aufbau in einer Längsschnittdarstellung.

Der Keramik-Grundkörper 15 besteht im Beispiel aus einer Aluminiumoxid-Keramik, kann aber etwa auch aus einer Zirkonoxid-Keramik oder einem anderen biokompatiblen Keramikmaterial mit herkömmlichen Techniken unter Aussparung von an die Form der Leiterelemente (Pins) 13 angepassten Öffnungen vorgefertigt sein. An bzw. in den Keramik-Grundkörper 15 sind der Umfangsflansch 17 und die Leiterelemente mittels des Metall-Pulverspritzgießens (MIM-Verfahren) hermetisch dicht an- bzw. eingeformt.

Eine kraftschlüssig dichte Verbindung zwischen den Teilen entsteht durch ein Schrumpfen der Metallteile in diesem Prozess. Um diesen Effekt für die Pins 13 zielführend nutzen zu können, sind diese an beiden Oberflächen des Keramik-Grundkörpers 15 jeweils mit ringförmigen Verdickungen 13a versehen. Diese pressen sich beim MIM-Prozess in Folge der Längen-Schrumpfung der Pins jeweils gegen die benachbarte innen liegende Ringfläche des Keramik-Grundkörpers 15 an und verbinden sich mit ihm sowohl kraftschlüssig als auch durch einen physiko-chemischen Grenzflächenprozess. Ein analoger Prozess läuft am Innenumfang des Umfangsflansches 17 gegenüber dem Außenumfang des Keramik-Grundkörpers 15 ab.

Die Pins 13 und der Umfangsflansch 17 können in einem gemeinsamen MIM-Prozessablauf aus dem gleichen Material (etwa einer Titanlegierung) gebildet werden, es ist aber auch eine Herstellung aus verschiedenen Materialien in aufeinanderfolgenden Prozessen möglich. Nach dem Entbindern und Sintern können die Pins mit Zusatzteilen (Hülsen oder Pads) oder geeigneten Beschichtungen für den Anschluss weiterer Elemente komplettiert werden, die in den Figuren nicht dargestellt, dem Fachmann aber an sich bekannt sind. Der Umfangsflansch 17 kann in üblicher Weise mit einem entsprechend verlaufenden Öffnungsrand des Gehäuses 3 (Fig. 1) verbunden werden, etwa durch ein Laserschweißverfahren.

Fig. 4 zeigt als weiteres Ausführungsbeispiel das distale Ende einer Elektrodenleitung 1', bei der eine Durchführung 11' das Leitungsende bildet. Die Durchführung 11' umfasst einen einzelnen Anschlussstift 13', einen Keramik-Grundkörper 15' und ein Umfangsflansch 17' und ist in das freie Ende der Leitungshülle 3' eingeschrumpft oder eingeklebt. Der Anschlussstift 13' ist in seinem leitungs-äußeren Abschnitt zugleich das äußere Funktionselement (Elektrode) der Elektrodenleitung und an seinem proximalen Ende in herkömmlicher Weise (etwa durch Weichlöten) mit einer elektronischen Funktionseinheit 7' verbunden. Bei der Ausführung nach Fig. 4 haben sowohl der Anschlussstift 13' als auch der Umfangsflansch 17' jeweils an den gegenüberliegenden Flächen des Keramik-Grundkörpers (Isolators) 15' ringförmige Verdickungen 13a' bzw. 17a', die in der weiter oben beschriebenen Weise im MIM-Prozess in Längsrichtung aufeinander zu schrumpfen und dazu führen, dass sich sowohl der Anschlussstift 13' als auch der Umfangsflansch 17' kraftschlüssig und dicht mit dem Isolator 15' verbinden.

Die Ausführung der Erfindung ist nicht auf diese Beispiele beschränkt, sondern ebenso in einer Vielzahl von Abwandlungen möglich, die im Rahmen fachgemäßen Handelns liegen.

## Patentansprüche

1. Implantierbare elektromedizinische Vorrichtung (1; 1'), umfassend ein
e elektronische oder elektrische Funktionseinheit (7; 7'),
ein Umhüllungsteil (3; 3'), das die elektronische oder elektrische Funktionseinheit (7; 7') aufnimmt, eine an der Außenseite des Umhüllungsteils angeordnete Elektrode oder einen Leitungsanschluss (9) und
eine Durchführung (11; 11'), die eine Öffnung des Umhüllungsteils umschließt und mindestens ein die Elektrode oder den Leitungsanschluss mit der Funktionseinheit verbindendes metallisches Leiterelement (13; 13') aufnimmt und einen metallischen Umfangsflansch (17; 17') hat,
wobei die Durchführung einen Isolator-Grundkörper (15; 15') aufweist, um den der Umfangsflansch und in den das Leiterelement direkt form- und kraftschlüssig verbindend geschrumpft ist, **dadurch gekennzeichnet, dass** der Umfangsflansch (17; 17') und das Leiterelement (13; 13') als materialeinheitlich durch Metall-Pulverspritzgießen gebildete Sinterteile ausgeführt sind.

2. Vorrichtung nach Anspruch 1, ausgebildet als Elektrostimulationsgerät (1), insbesondere Herzschrittmacher oder Kardioverter, wobei das Umhüllungsteil als Gerätegehäuse (3) ausgebildet und die Elektrode oder der Leitungsanschluss an einem gegenüber dem Gerätegehäuse separaten Kopfteil (5) angeordnet ist und die Durchführung (11) zwischen dem Gerätegehäuse und dem Kopfteil angeordnet ist.

3. Vorrichtung nach Anspruch 1, ausgebildet als Elektrodenleitung (1'), wobei insbesondere das Leiterelement (13') in seinem umhüllungs-äußeren Abschnitt zugleich eine Elektrode bildet.

4. Vorrichtung nach einem der vorangehenden Ansprüche, wobei am Umfangsflansch (17; 17') oder dem Leiterelement (13; 13') mindestens ein sich in eine korrespondierende Ausnehmung des Isolator-Grundkörpers (15; 15') erstreckender Fortsatz (13a; 13a'; 17a') vorgesehen ist.

5. Vorrichtung nach Anspruch 4, wobei am Umfangsflansch (17) und dem oder jedem Leiterelement (13) je ein derart direkt an einer vorrichtungs-inneren und einer vorrichtungs-äußeren Oberfläche oder einer dort angeordneten Ausnehmung des Isolator-Grundgehäuses (15) angeordneter Fortsatz (13a) vorgesehen ist, dass der äußere und der innere Fortsatz fest an die jeweilige Oberfläche des Isolator-Grundkörpers angepresst sind.

6. Vorrichtung nach Anspruch 4 oder 5, wobei der oder jeder Fortsatz (13a; 13a'; 17a') ring- oder scheibenförmig den Innenumfang des Umfangsflansches (17; 17') bzw. den Außenumfang des Leiterelements (13; 13') umgebend ausgebildet ist.

7. Vorrichtung nach einem der vorangehenden Ansprüche, wobei der Isolator-Grundkörper (15; 15') im Wesentlichen aus Aluminiumoxid-Keramik gebildet ist.

8. Vorrichtung nach einem der vorangehenden Ansprüche, wobei der Umfangsflansch (17; 17') und/oder das Leiterelement (13; 13') aus Titan oder einer Titanlegierung gebildet sind.

9. Verfahren zur Herstellung einer Vorrichtung nach einem der vorangehenden Ansprüche, mit den Schritten:
- Bilden des Isolator-Grundkörpers (15; 15'),
- Bereitstellen einer Pulverspritzgussform,
- Einlegen des Isolator-Grundkörpers in teilweise oder vollständig vorgesintertem Zustand in die Pulverspritzgussform,
- Einspritzen von Spritzgießmaterial zur Bildung des Umfangsflansches (17; 17') und des oder jedes Leiterelementes (13; 13') in die Pulverspritzgussform,
- Sintern/Wärmebehandlung der Pulverspritzgussform mit dem darin aufgenommenen Material zum Entbindern, Ausgasen und Schrumpfen des den Umfangsflansch und das oder jedes Leiterelement bildenden Materials und
- Entnehmen der Durchführung (11) aus der Pulverspritzgussform.

10. Verfahren nach Anspruch 9, mit dem späteren Schritt eines Verbindens der Durchführung (11; 11') mit der Umhüllung (5; 5'), insbesondere durch Verschweißen oder Verkleben oder Aufschrumpfen der Umhüllung auf den Umfangsflansch (17; 17') der Durchführung (11; 11').

## Claims

1. An implantable electromedical device (1; 1'), comprising:
an electronic or electric functional unit (7; 7');
a casing part (3; 3') which accommodates the electronic or electric functional unit (7; 7');
an electrode, arranged on the outside of the casing part, or a lead connection (9); and
a feedthrough (11; 11'), which surrounds an opening of the casing part and accommodates at least one metallic conductor element (13; 13') connecting the electrode or the lead connection to the functional unit and which has a metallic circumferential flange (17; 17'),
wherein the feedthrough comprises an insulator main body (15; 15'), around which the circumferential flange and in which the conductor element are directly shrunk in a form-fitting and force-fitting connecting manner, **characterised in that** the circumferential flange (17; 17') and the conductor element (13; 13') are designed as sintered parts formed of the same material by way of metal injection moulding.

2. The device according to claim 1, designed as an electrostimulation device (1), in particular cardiac pacemaker or cardioverter, wherein the casing part is designed as a device housing (3), and the electrode or the lead connection (9) is arranged on a header (5), which is separate from the device housing, and the feedthrough (11) is arranged between the device housing and the header.

3. The device according to claim 1, designed as an electrode lead (1'), wherein in particular the conductor element (13') in its casing-outer portion at the same time forms an electrode.

4. The device according to any one of the preceding claims, wherein at least one extension (13a; 13a'; 17a') extending in a corresponding cut-out of the insulator main body (15; 15') is provided on the circumferential flange (17; 17') or the conductor element (13; 13').

5. The device according to claim 4, wherein an extension (13a), which is arranged directly on a device-inner and a device-outer surface or on a cut-out of the insulator main housing (15) provided there, is provided on the circumferential flange (17) and on the, or each, conductor element (13), such that the outer and inner extensions are pressed securely against the corresponding surface of the insulator main body.

6. The device according to claim 4 or 5, wherein the, or each, extension (13a; 13a'; 17a') is designed to surround the inner circumference of the circumferential flange (17; 17') or the outer circumference of the conductor element (13; 13') in a ringshaped or disc-shaped manner.

7. The device according to any one of the preceding claims, wherein the insulator main body (15; 15') is formed substantially of aluminium oxide ceramic.

8. The device according to any one of the preceding claims, wherein the circumferential flange (17; 17') and/or the conductor element (13; 13') are/is formed of titanium or a titanium alloy.

9. A method for producing a device according to any one of the preceding claims, said method having the following steps:
- forming the insulator main body (15; 15');
- providing a metal injection mould;
- inserting the insulator main body in the partially or fully pre-sintered state into the metal injection mould;
- injecting moulding material into the metal injection mould to form the circumferential flange (17; 17') and the, or each, conductor element;
- sintering/heat treating the metal injection mould with the material accommodated therein for debinding, outgassing and shrinking the material forming the circumferential flange and the or each conductor element; and
- removing the feedthrough (11) from the metal injection mould.

10. The method according to claim 9, comprising the later step of connecting the feedthrough (11; 11') to the casing (5; 5'), in particular by welding or bonding or shrink fitting the casing to the circumferential flange (17; 17') of the feedthrough (11; 11').

## Revendications

1. Dispositif électromédical implantable (1 ; 1'), comprenant
une unité fonctionnelle (7 ; 7') électronique ou électrique,
une partie d'enveloppe (3 ; 3') qui reçoit l'unité fonctionnelle (7 ; 7') électronique ou électrique,
une électrode disposée sur le côté externe de la partie d'enveloppe, ou une connexion électrique (9), et
un passage (11 ; 11'), qui entoure un orifice de la partie d'enveloppe et reçoit au moins un élément conducteur (13 ; 13') métallique reliant l'électrode ou la connexion électrique avec l'unité fonctionnelle et qui a une bride périphérique (17 ; 17') métallique,
où le passage présente un corps de base d'isolateur (15 ; 15') autour duquel la bride périphérique et dans lequel l'élément conducteur sont contractés en étant reliés directement par complémentarité des formes et des matières, **caractérisé en ce que** la bride périphérique (17 ; 17') et l'élément conducteur (13 ; 13') sont conçus d'un seul tenant par des pièces frittées formées par un moulage par injection de poudre métallique.

2. Dispositif selon la revendication 1, conçu sous forme d'appareil d'électrostimulation (1), notamment de stimulateur cardiaque ou de cardioverteur, où la partie d'enveloppe est conçue sous forme de boîtier d'appareil (3) et l'électrode, ou la connexion électrique, est disposée sur une partie frontale (5) séparée par rapport au boîtier d'appareil et le passage (11) est disposé entre le boitier d'appareil et la partie frontale.

3. Dispositif selon la revendication 1, conçu sous forme de ligne d'électrode (1'), dans lequel, notamment, l'élément conducteur (13') dans sa section extérieure à l'enveloppe forme simultanément une électrode.

4. Dispositif selon l'une des revendications précédentes, dans lequel au moins une extension (13a ; 13a' ; 17a') s'étendant dans une cavité correspondante du corps de base d'isolateur (15 ; 15') est prévue sur la bride périphérique (17 ; 17') ou sur l'élément conducteur (13 ; 13').

5. Dispositif selon la revendication 4, dans lequel une extension (13a) disposée directement, respectivement sur une surface intérieure de dispositif et sur une surface extérieure de dispositif, ou dans une cavité du boitier de base d'isolateur (15), est prévue sur la bride périphérique (17) ou sur l'élément ou chaque élément conducteur (13), de sorte que l'extension extérieure et l'extension intérieure sont pressées solidement contre la surface respective du corps de base d'isolateur.

6. Dispositif selon la revendication 4 ou la revendication 5, dans lequel l'extension ou chaque extension (13a; 13a' ; 17a') est conçue en forme d'anneau ou de disque entourant le périmètre intérieur de la bride périphérique (17 ; 17'), respectivement, le périmètre extérieur de l'élément conducteur (13 ; 13').

7. Dispositif selon l'une des revendications précédentes, dans lequel le corps de base d'isolateur (15 ; 15') est formé essentiellement à base d'une céramique à base d'oxyde d'aluminium.

8. Dispositif selon l'une des revendications précédentes, dans lequel la bride périphérique (17 ; 17') et/ou l'élément conducteur (13 ; 13') sont formés à base de titane ou d'un alliage de titane.

9. Procédé de fabrication d'un dispositif selon l'une des revendications précédentes, avec les étapes :
- de formation du corps de base d'isolateur (15 ; 15'),
- de fourniture d'un moule pour un moulage par injection de poudre,
- de dépôt du corps de base d'isolateur à l'état partiellement ou totalement préfritté dans le moule pour le moulage par injection de poudre,
- d'injection de la matière de moulage par injection pour la formation de la bride périphérique (17 ; 17') ou de l'élément ou de chaque élément conducteur (13 ; 13') dans le moule pour le moulage par injection de poudre,
- de frittage/traitement thermique du moule pour le moulage par injection de poudre avec le matériau y étant réceptionné pour le déliantage, le dégazage et la contraction du matériau formant la bride périphérique et l'élément ou chaque élément conducteur, et
- de réalisation du passage (11) à partir du moule pour le moulage par injection de poudre.

10. Procédé selon la revendication 9, avec l'étape ultérieure d'une liaison du passage (11 ; 11') avec l'enveloppe (5 ; 5'), notamment par un soudage ou un collage, ou une contraction de l'enveloppe par-dessus la bride périphérique (17 ; 17') du passage (11 ; 11').
